# EUROPEAN PATENT APPLICATION

(11) **EP 3 156 587 A1**
(43) Date of publication of application: **19.04.2017**
(21) Application number: 15290263.1
(22) Date of filing: 16.10.2015
(51) Int. Cl.: E21B 49/00, G01N 1/28, G01N 33/24

(54) **AUTOMATED DEVICE FOR THE ANALYSIS OF DRILLING CUTTINGS**

(71) Applicant: Services Pétroliers Schlumberger, 75007 Paris (FR); Schlumberger Holdings Limited, Road Town, Tortola (VG); Schlumberger Technology B.V., 2514 JG The Hague (NL)
(72) Inventor: Kimour, Farouk, 92140 Clamart (FR); Mansour, Darine, 92140 Clamart (FR)
(74) Representative: Leonori, Céline

(57) **Abstract**

The disclosure relates to an automated device for analyzing drilling cuttings, the device comprising:
- a sampler for sampling drilling cuttings; -

an analysis unit for analyzing the treated cuttings;

wherein the analysis unit comprises:
- a preparation apparatus able to grind the treated cuttings into a powder and a pelletizer to compact the powder into a pellet for X-ray analysis;

- an analyzer;

and wherein the automated device comprises a transport unit for moving the analysis sample from the sampler to the analyzer, and a control device configured to trigger the operation of each of the units according to a predetermined sequence.

## Description

The present disclosure relates to an automated device for analyzing drilling cuttings, said device comprising a sampler for sampling drilling cuttings and an analysis unit for analyzing the treated cuttings.

### BACKGROUND

During the drilling process of an oil well or of a well of another effluent - In particular gas, vapour or water -, it is known to carry out a continuous analysis of the rock cuttings brought to the surface by a circulating drilling medium, especially by a drilling mud. Such analysis, known as mud logging, allows the creation of a detailed record of the geologic formations of a borehole, in function of the well bore depth.

The quality of mud logging analyses relies particularly on the representativeness of the cuttings, on the quality of the sample preparation and on the level of cross-contamination between the samples. Moreover, the mud logging environment is unsafe for human operators and the mud logging activities are tedious.

The automation of some mud logging steps, such as the sampling and washing of the cuttings and certain types of analysis, is described in known documents, such as US6386026 and US2692755.

Some of the analysis techniques used in mud logging, such as the ones related to X-ray analysis, in particular X-ray diffraction (XRD) analysis or X-ray fluorescence (XRF) analysis, require a specific preparation for the cuttings, including the grinding of the cuttings into a powder and the conformation of said powder into a sample. Usually, these preparation steps are carried out by a human operator,

### SUMMARY OF THE DISCLOSURE

The present disclosure relates to an automated device comprising a sampler for sampling drilling cuttings and an analysis unit for analyzing the treated cuttings. The analysis unit comprises : a preparation apparatus for grinding the treated cuttings into a powder and conform said powder into an analysis sample; and analyzers.The automated device also comprises a transport unit for moving the analysis sample from the sampler to the analyzer ; and a control device to trigger at least an operation of each of the units according to a predetermined sequence.

Such a device allows a complete automation of the mud logging process, without any operator intervention. The human factor is thereby reduced and the operating costs are improved.

According to embodiments, the automated device comprises one or more of the following features, taken in isolation or In any technically possible combinaiton(s):
- the control device is configured to trigger at least an operation of at least a predetermined unit in function of a state of at least one of the units and/or of an external parameter; the state of at least one of the units may be the state of the predetermined unit, or of any other unit such as the unit performing a previous or following operation in the sequence ; the external parameter may be a movement of the rig, the control device compensating for the movement;
- the automated device comprises at least one sensor for detecting a state of one of the units / external parameter ; the sensor may be chosen among a position sensor, weighing sensor or accelerometer;
- the transport unit comprises a robotic manipulator able to insert the sampled cuttings into the preparation apparatus and/or to remove the analysis sample from the at least one analyzer ;
- the control device is configured to command the robotic manipulator to insert the sampled cuttings into the preparation apparatus and/or to remove the analysis sample from the at least one analyzer;
- the analysis unit comprises shelves on which are arranged the preparation apparatus and the at least one analyzer, the robotic manipulator also comprising a gripper assembly and a gantry on which the gripper assembly is able to move between the shelves ;
- the at least one analyzer is an X-ray analyzer, a spectrometer or a TOC analyzer;
- the X-ray analyzer is chosen among an X-ray diffraction analyzer and an X-ray fluorescence analyzer;
- the spectrometer is chosen among a DRIFTS (Diffuse Reflectance infrared Fourier Transform Spectrometry) spectrometer, a Raman spectrometer, a LIBS (Laser induced Breakdown Spectrometry) spectrometer, and a ATR (Attenuated Total Reflectance) spectrometer;
- the analysis unit comprises at least two analyzers, the preparation apparatus being able to prepare an analysis sample suitable for each of said analyzers;
- the analysis unit comprises at least one additional analyzer, wherein the control device is configured to provide cuttings to the additional analyzer, said cuttings not passing through the grinding apparatus. The additional analyzer may be for instance a Scanning Electron Microscope (SEM) or a calcimeter.
- the preparation apparatus is able to add a binder to the cutting or to the powder to prepare the analysis samples for analysis;
- the preparation apparatus includes a mixer for mixing the binder and cutting or powder and/or a pelletizer ;
- the analysis unit may also comprise a drying apparatus and/or an imaging apparatus, the transport unit being able to move the sampled cuttings from said drying apparatus and/or imaging apparatus to the preparation apparatus;
- the control device is configured to command the robotic manipulator to move the sampled cuttings from said drying apparatus and/or imaging apparatus to the preparation apparatus;
- the automated device comprises a treatment unit for treating the sampled cuttings;
- the transport unit is configured to transport the cuttings from the sampler to the treatment unit and from the treatment unit to the analysis unit;
- the control device is configured to command the robotic manipulator to transport the cuttings from the sampler to the treatment unit and from the treatment unit to the analysis unit ;
- the treatment unit comprises : a washing apparatus able to remove drilling mud from the sampled cuttings, and/or an archiving apparatus able to archive the sampled cuttings;
- the transport unit comprises at least a pneumatic tube transfer apparatus and/or at least a conveyor ;
- the automated device comprises a weighing unit for weighing the sample ;
- the transport device is configured to transport cuttings from the weighing device to the analysis unit;
- the control device is configured to command the robotic manipulator to transport cuttings from the weighing device to the analysis unit.

The present disclosure also relates to a drilling installation for drilling a wellbore, comprising : a shale shaker situated at the exit of the wellbore for separating a drilling fluid exiting the wellbore and drilling cuttings contained in the drilling fluid, and an automated device for analyzing drilling cuttings, as described above, said automated device being situated at the outlet of the shale shaker.

### BRIEF DESCRIPTION OF THE DRAWINGS

The present disclosure will be better understood upon reading the following description, which is given solely by way of example, and which is written with reference to the appended drawings, in which:
- Figure 1 is a schematic view, in vertical section, of a drilling installation provided with an automated device according to an embodiment of the present disclosure ;
- Figure 2 is a schematic view, from above, of a first part of the automated device of Figure 1 ;
- Figure 3 is a schematic side view of a second part of the automated device of Figure 1 ;
- Figure 4 is a schematic side view of a third part of the automated device of Figure 1 according to an embodiment of the disclosure; and
- Figure 5 is a schematic, perspective view of a third part of the automated device according to another embodiment of the disclosure, according to an alternative embodiment.

### DETAILED DESCRIPTION

An automated device 10 for analyzing drilling cuttings, as described above, may be included in a drilling installation 12 for a fluid production well, such as a hydrocarbon production well.

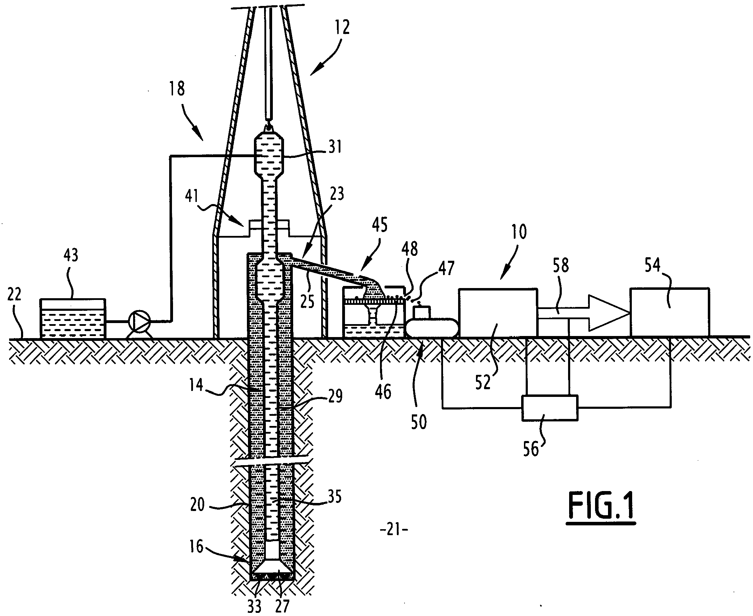

Such an installation 12, illustrated on Figure 1, comprises: a rotary drilling tool 14 drilling a cavity 16 ; a surface installation 18, where drilling pipes are placed In the cavity 16 ; and the automated device 10 described above.

A borehole 20, delimiting the cavity 16, is formed in the substratum 21 by the rotary drilling tool 14. At the surface 22, a well head 23 having a discharge pipe 25 closes the borehole 20.

The drilling tool 14 comprises a drilling head 27, a drill string 29 and a liquid injection head 31.

The drilling head 27 comprises a drill bit 33 for drilling through the rocks of the substratum 21. It is mounted on the lower portion of the drill string 29 and is positioned in the bottom of the drilling pipe 20.

The drill string 29 comprises a set of hollow drilling pipes. These pipes delimit an internal space 35 which makes it possible to bring a drilling fluid from the surface 22 to the drilling head 27. To this end, the liquid injection head 31 is screwed onto the upper portion of the drill string 29.

The drilling fluid is a drilling mud, in particular a water-based or oil-based drilling mud.

The surface installation 18 comprises a support 41 for supporting the drilling tool 14 and driving it in rotation, an injector 43 for injecting the drilling fluid and a shale shaker 45.

The injector 43 is hydraulically connected to the injection head 31 in order to introduce and circulate the drilling fluid in the inner space 35 of the drill string 29.

The shale shaker 45 collects the drilling fluid charged with drilling residues, known as cuttings, said drilling fluid flowing out from the discharge pipe 25. The shale shaker comprises a sieve 46 allowing the separation of the solid drilling cuttings 47 from the drilling mud. The shale shaker 45 also comprises an outlet 48 for evacuating the drilling cuttings 47.

The automated analyzing device 10 according to an embodiment of the disclosure and situated at the outlet of the shale shaker 45 comprises: a sampler 50 for sampling drilling cuttings from the outlet 48 of the shale shaker 45 ; a treatment unit 52 for operations such as washing the sampled cuttings ; and an analysis unit 54 for analyzing the sampled cuttings. The automated analyzing device 10 also comprises a control device 56, connected to the sampler 50, treatment unit 52 and analysis unit 54. The automated analyzing device 10 may also comprises a transport unit 58 for moving the sampled cuttings from the sampler 50 to the analysis unit 54, said transport unit being connected to the control device 56, such as each of the sampling, treatment and analysis unit. The device 10 according to another embodiment of the disclosure may not comprise the treatment unit 52.

The control device may comprise a central controller and/or several local controllers, for Instance a controller for each unit. The automated device may store, associated with the control device, at least an operations sequence. For each sequence, an order of the operations performed by each unit is determined. An operations sequence may comprise several units performing operations subsequently and/or in parallel and may be optimized to ensure an analysis time as short as possible.

The control device is configured to trigger each unit in function of at least a predetermined sequence. It may also be configured to trigger at least a unit in function of the state of at least one of the units. The control device may for instance take into account the state of the unit it triggers, and/or the state of one other unit. The state of each unit may be determined via sensors such as a position sensor, weighing sensor, etc. The control device may for instance be configured to trigger an operation of a unit in function of the state of the unit performing the preceding operation - In order to ensure the preceding operation has been performed correctly - and/or the state of the unit It triggers - in order to ensure the unit is ready to perform the operation - and/or the state of the unit performing the following operation. For instance, the control device may be configured to trigger the sampler and, when it detects the sampler has performed the sampling operation, to trigger the transport unit for transporting the cuttings to the preparation apparatus of the analysis unit. When it detects the cuttings are in a certain position relative to the preparation apparatus, the control device may trigger the preparation apparatus and when it detects the preparation apparatus has performed the preparation operation or after a certain amount of time, It triggers the transport unit for transporting the cuttings to the analyzer and then triggers the analyzer when it detects the cuttings are in a certain position relative to the analyzer. If there is a treatment unit, it may be triggered before the preparation apparatus, the preparation apparatus being triggered only when the treatment operation is over.

The device may also measure external parameters such as a movement of a rig platform on which the automated device is installed. The control device may then be configured to trigger at least one of the unit, in function of the measured external parameter. The control device may then take into account the effect of the platform motion on the different operations and compensate it in order to be able to perform the analysis efficiently even in a harsh environment; for example the positioning of transport unit (like the manipulator) may be affected by inertial forces due to platform motion; in this case, the control algorithms like the positioning control compensate or reject these inertial forces to ensure a robust and precise positioning. As another example, when the platform is not moving, the control device may trigger a first operation (movement) of the transport unit but, when the platform is moving, it may delay the operation or trigger a second operation (movement) of the transport unit.

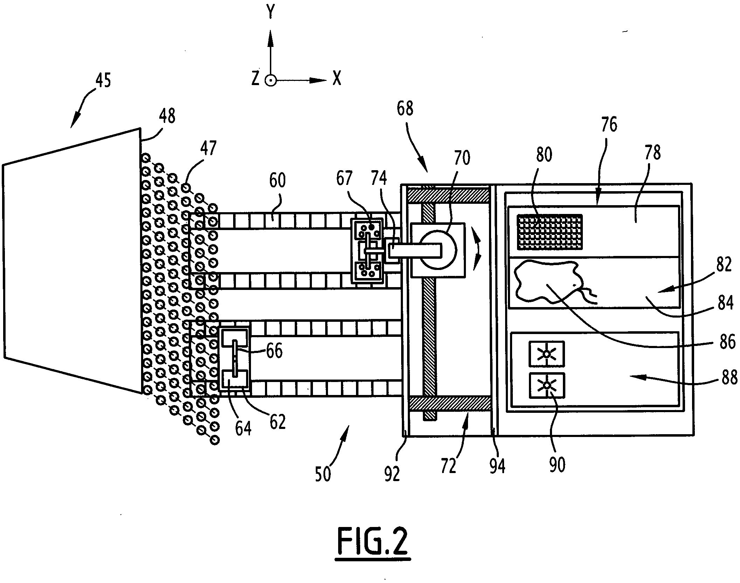

Figure 2 shows a top view of a part of the drilling Installation 12 according to an embodiment of the disclosure. More specifically, Figure 2 shows the shale shaker 45 and the sampler 50 and treatment unit 52 of the automated analyzing device 10.

The sampler 50 comprises at least one conveyor 60 disposed between the outlet 48 of the shale shaker 45 and the treatment unit 52. In the embodiment of Figure 2, the sampler 50 comprises two conveyors 60 parallel to each other. The conveyors 60 are able to move back and forth along a horizontal direction X, part of an orthonormal basis (X, Y, Z).

The sampler 50 comprises at least one canister 62 able to be carried back and forth along the direction X by the at least one conveyor 60. In the embodiment of Figure 2, said canister 62 comprises two cavities 64 and a handle 66 between said cavities. In the embodiment of Figure 2, the sampler 50 comprises two canisters 62, for collecting cuttings continuously.

A first end along X of the conveyors 60 is situated under the outlet 48, so that, when the canisters 62 are situated near said first end, drilling cuttings 47 fall into the cavities 64. Said cuttings 47 fallen into a cavity 64 constitute sampled cuttings 67. A second end along X of the conveyors 60 is situated next to the treatment unit 52.

In an alternative embodiment (not shown), the conveyors 60 are situated on each side of the outlet 48, and each conveyor comprises an arm extending a canister 62 under said outlet 48. Such an embodiment lessens the amount of cuttings 47 susceptible to fall directly on the conveyors 60.

The treatment unit 52 comprises a handling device 68, situated next to the second end along X of the conveyors 60. In the embodiment of Figure 2, the handling device 68 comprises a robotic manipulator including a robotic arm 70 and a gantry 72. The robotic arm 70 Is able to move along two horizontal axes (X, Y) on the gantry 72. The robotic arm 70 comprises a gripper 74 able to seize the handle 66 of the canister 62. The treatment unit 52 also comprises a washing station 76 able to wash the sampled cuttings 67. The washing station 76 comprises for Instance a conveyor belt 78 able to move along X. A first end 79 along X of the conveyor belt 78 is situated next to the handling device 68, opposite to the conveyors 60. The washing station 76 also comprises a tray 80 with wire-mesh bottom. The tray 80 is attached to the first conveyor belt 78 and moved along X by said conveyor belt.

The washing station 76 will be more specifically described below.

In the embodiment of Figure 2, the treatment unit 52 also comprises an archiving station 82 able to archive the sampled cuttings 67. The archiving station 82 comprises an automatic device (not shown) able to close, tag and store an archiving bag 86, such as an automatic bag sealing and trimming with inline label printing.

In the embodiment of Figure 2, the treatment unit 52 also comprises a canister cleaning station 88. The cleaning station 88 comprises two jet nozzles 90 able to propel high pressure fluid jets into the cavities 64 of a canister 62. The jet nozzles 90 are next, along Y, to the first ends of the first 78 and of the second 84 conveyor belts. The canisters, once cleaned are then able to go back to the sampler 50. Same canister may be used several times without cross-contamination of the sample with cuttings or residues of a sample taken previously.

In order to separate the washing 76 and archiving 82 stations from the sampler 50, the treatment unit 52 also comprises a first trap door 92, situated between the conveyors 60 and the handling device 68, and/or a second trap door 94, situated between the handling device 68 and the washing 76, archiving 82 and canister cleaning 88 stations.

In another embodiment of the sampler, the conveyors 60 may be replaced by a ball screw linear module carrying two sample canisters and moving back and forth along the shaker exit during the collection time. This embodiment avoids loss of cuttings and thus, loss of representativeness. After the collection time is up, the module delivers the canisters to the treatment unit 52 described above.

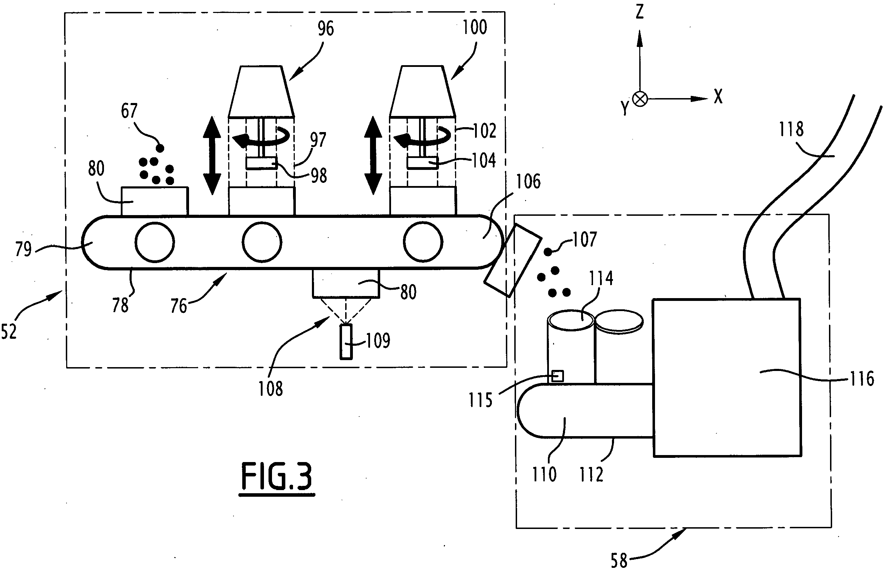

Figure 3 shows a side view of a part of the treatment unit 52 and of a part of the transport unit 58, able to transport cuttings from the treatment unit to the analysis unit.

In particular, Figure 3 shows a side view of the washing station 76, and especially of the conveyor belt 78 and of the tray 80.

In the example of Figure 3, the conveyor belt 78 is an over/under conveyor belt, on which the tray 80 is attached. Above the conveyor belt 78 is situated a rinsing device 96, near the first end 79 of said conveyor belt 78. The rinsing device 96 is able to pour base fluid 97 on the sampled cuttings 67 contained in the tray 80, in order to remove the drilling fluid (or mud). The rinsing device 96 also comprises a stirrer 98 to stir the cuttings during rinsing. The rinsing device is optional and may not be used for instance in the case the drilling fluid is water-based mud.

Next to the rinsing device 96, along X, is situated a washing device 100. The washing device 100 is able to pour one or several washing fluids 102 on the cuttings contained in the tray 80, to remove the traces of base fluid, such as an organic solvent or water with detergent, optionally followed by water. The washing device 100 also comprises a stirrer 104 to stir the cuttings during washing.

At a second end 106 along X of the washing station 76, the conveyor belt 78 is able to tilt the tray 80 by moving upside down, the tray being thereby emptied of the treated cuttings 107, Under the conveyor belt 78 is situated a tray cleaning device 108, comprising a nozzle 109 able to propel a fluid jet into the empty tray 80, which also avoids cross-contamination of the treated sample with a sample that was taken previously.

Any other example of treatment unit may be used: for instance, treatments units are known from patents or applications US2692755, U$6836026 or US2012/0234360

Under the second end 106 of the washing station 76 is situated a first end 110 of a transport device 58. At said first end 110, a conveyor 112 supports a carrier 114 able to receive the treated cuttings 107 discharged from the tray 80. The carrier 114 may bear an identification tag 115.

According to an alternative embodiment, the transport device 58 comprises a vibrating dispenser (not shown), situated at the first end 110 and able to receive the treated cuttings 107; the carrier 114 is situated on a scale (not shown) connected to said vibrating dispenser, so that said vibrating dispenser is able to pour a requested weight of cuttings 107 into the carrier 114.

The conveyor 112 is able to move the carrier 114 into an automated unit 116 able to close the carrier and to load said carrier into a sending pneumatic tube station 118. The pneumatic tube station 118 is connected to the analysis unit 54.

Alternatively, a robotic gripper assembly of automated unit 116 may directly grab the carrier 114 to the sending pneumatic tube station 118. A transport device 58 with a pneumatic tube station 118 may be used in the case of a significant distance between the treatment unit 52 and the analysis unit 54. As an example, the treatment unit 52 is situated next to the shale shaker 45 and the analysis unit 54 is situated on a different location, possibly distant of several hundred meters from the treatment unit 52.

The transport device 58 may also comprise a system (not shown) able to clean and re-use the carrier 114 after said carrier has reached the analysis unit 54. The analysis unit may however be situated near the shale shaker as well.

Other transport devices 58, such as a conveyor belt, telescopic conveyor, a manipulator, may be used, especially In the case of an analysis unit 54 situated near the treatment unit 52.

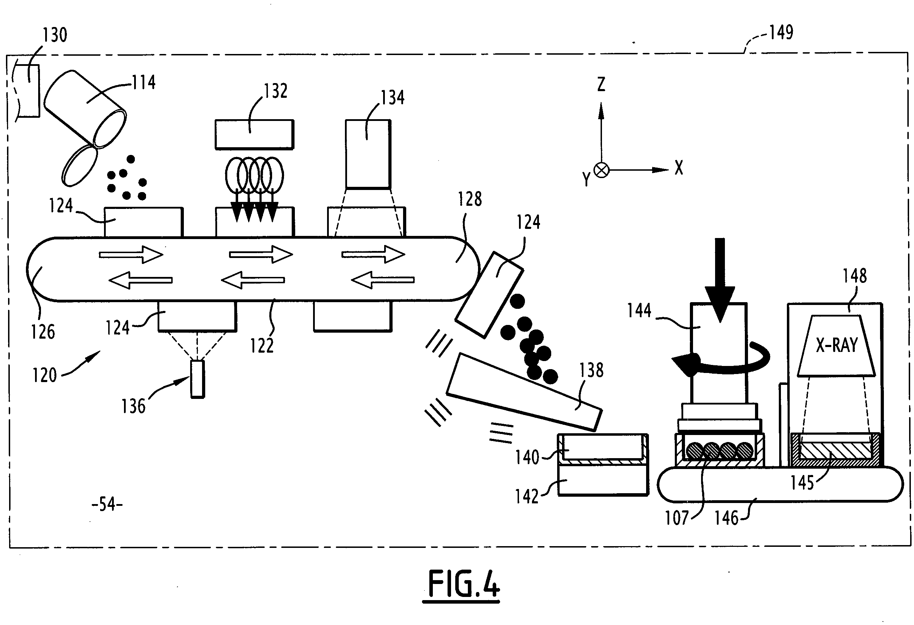

Figure 4 shows a schematic view of the analysis unit 54 according to an embodiment of the disclosure.

The analysis unit 54 comprises a drying and imaging station 120. In the embodiment of Figure 4, the drying and imaging station 120 comprises an over/under conveyor belt 122 and a tray 124, for instance with a wire-mesh bottom, attached to said conveyor belt In this embodiment, the conveyor belt 122 and the tray 124 are similar to the conveyor belt 78 and tray 80 of the washing station 76.

The conveyor belt 122 is able to move the tray 124 along a horizontal axis, between a first end 126 and a second end 128. In the embodiment of Figure 4, said horizontal axis is X.

At a second end 130 of the transport device 58, the analysis unit 54 comprises a robotic manipulator (not shown) able to open the carrier 114 and to pour the treated cuttings 107 into the tray 124 situated at the first end 126. Above the conveyor belt 122, next to said first end 126, the drying and imaging station 120 comprises a drying apparatus 132, able to dry the treated cuttings 107 contained in the tray 124. The drying apparatus 132 may be a fan or a heat lamp. Any other drying apparatus may be used for drying the cuttings. Next to the drying apparatus 132, the drying and imaging station 120 comprises an imaging apparatus 134 such as a digital camera or a digital microscope. Alternatively, the imaging apparatus may be disposed before the drying apparatus.

At the second end 128. the conveyor belt 122 is able to tilt the tray 124 by moving upside down, the tray being thereby emptied of the cuttings 107. Under the conveyor belt 122 is situated a tray cleaning device 136, comprising for instance a nozzle able to propel a fluid jet or pressurized air into the empty tray 124.

Alternatively, the drying or imaging apparatuses may be implemented on the same conveyor than the washing and/or rinsing device.

Under the second end 128 of the conveyor belt 122 is situated a vibrating dispenser 138, able to pour the cuttings 107 into at least one pellet die 140 supported by a scale (or weighing unit) 142. The vibrating dispenser 138 is connected to the scale 142, directly or through the control device 56, so that the vibrating dispenser 138 stops when the pellet die 140 has received a sufficient weight of cuttings 107.

The analysis unit 54 also comprises a mechanized system such as a pusher able to move the pellet die 140 full of cuttings 107 into a preparation apparatus 144. The preparation apparatus 144 is able to crush the treated cuttings 107 into a powder and subsequently to conform said powder into an analysis sample for X-ray analysis. As an example, the preparation apparatus 144 comprises a grinding device to grind the cuttings 107 and a pellet press (or pelletizer) to compact the resulting powder into a pellet 145.

Optionally, the preparation apparatus 144 comprises a sieve (not shown) to control the powder grain size resulting from the grinding.

Optionally, the preparation apparatus 144 comprises a device (not shown) for adding a binder to the powder before grinding and compacting said powder. As an example, the binder is a wax. The preparation apparatus 144 may also comprise a mixer (not shown) to obtain an homogeneous blend from the powder and the binder. The mixed blend will be compacted into a pellet.

The analysis unit 54 also comprises a conveyor 146 able to move the pellet 145 to an X-ray analyzer 148. The X-ray analyzer 148 may be an X-ray diffraction (XRD) analyzer, or the X-ray analyzer 148 may be an X-ray fluorescence (XRF) analyzer. After the X-ray analysis is completed, the conveyor 146 is able to discard the pellet 145.

Optionally, the analysis unit 54 comprises two X-ray analyzers 148, one of them being a XRD analyzer and the other being a XRF analyzer. The analysis unit 54 also comprises two preparation apparatus 144, each of them being able to prepare an analysis sample suitable for one of the X-ray analyzers 148. The vibrating dispenser 138 is able to pour cuttings 107 into two pellet dies 140, each of them supported by a scale 142, so that the two X-ray analyses may be carried out simultaneously.

The analysis unit 54 is a fully integrated unit, enclosed in a safety cage 149 with shut-off safety interlock. The integrated unit may also include devices such as the imaging or drying device and even devices of the treatment unit.

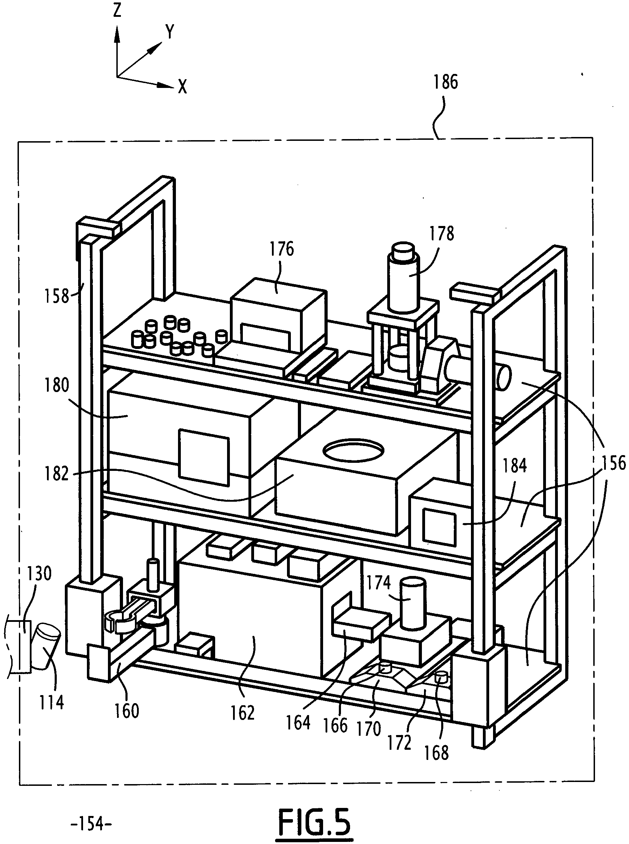

Figure 5 shows a schematic view of an analysis unit 154 according to an alternative embodiment of the disclosure. The analysis unit 154 is able to replace the analysis unit 54 in the automated device 10 of Figure 1.

The analysis unit 154 comprises different elements distributed on at least two shelves 156. The shelves 156 are arranged vertically in order to occupy a minimum base surface. The shelves 156 are attached to a vertical gantry 158. In the embodiment of Figure 5, the gantry 158 is situated in a (X, Z) plane. The shelves may also comprise devices of the treatment unit, such as the washing, rinsing or archiving device.

A robotic manipulator comprises a gripper assembly (robotic gripper and wrist) 160 and a gantry 158, the gripper assembly being able to move along the gantry 158. The gripper assembly 160 comprises a gripper able to seize the carrier 114 arriving at the second end 130 of the transport unit 58, to open the carrier 114 and to empty It into a tray of a drying and imaging station 162. Said drying and imaging station 162 is similar to the drying and imaging station 120 of Figure 4..

The gripper assembly 160 is able to remove the cuttings 107 from an outlet 164 of the drying and imaging station 162, and to pour some of said cuttings into two grinding canisters 166, 168, each of them being supported by a scale 170, 172. The feedback readings of scales 170,172 allow said gripper assembly put a suitable amount of cuttings 107 on each canister 166,168, depending on the requested analysis described below.

Above one 170 of the scales may be situated an automated dispenser 174 able to add a wax binder into one 166 of the canisters.

The analysis unit 154 also comprises a ball mill 176, a pellet press 178, an XRD analyzer 180 and a XRF analyzer 182. The analysis unit 154 also comprises a cleaning station 184. These devices are arranged on the shelves 156, accessible by the gripper assembly 160 through the gantry 158.

The analysis unit 154 may be enclosed in a safety cage 186, with shut-off safety interlock.

According to an alternative embodiment, the analysis unit 154 comprises two or more gripper assemblies 160 able to move on the same gantry 158, or in different gantries, to operate simultaneously.

A manipulator with alternative architecture (for instance a robotic arm, not movable relative to a gantry such as a joint-arm robot) may also be used for transporting the samples.

An operation method of the automated device 10 as part of the installation 12 will now be described, with the embodiment 154, shown on Figure 5, of the analysis unit Such a method would be similar with the analysis unit 54 of Figure 4.

The electronic control device 56 receives information from a processing system controlling the drilling tool 14 ; said information relates for example to the current drilled depth, the current drilling rate of penetration, the lag depth between the drill bit 33 and the shale shaker 45. The electronic control device 56 may also be provided with information such as the sampling depth interval required by a customer.

In function of these parameters, the control device 56 determines a suitable amount of time between two consecutive samplings by the sampler 50. In other terms, the control device 56 determines a suitable frequency of courses for the conveyors 60, between the outlet 48 of the shale shaker 45 and the treatment device 52, and commands the sampler in function of this information. In the embodiment of Figure 2. when a large amount of time is required between two consecutive samplings, only one conveyor 60 may be used.

The amount of cuttings 67 of each sampling is determined by the inner volume and opening dimensions of each canister 62.

When it is detected that the canister is full, or after a certain sampling time, the control device triggers the conveyor so that it transports the sampled cuttings to the treatment device 52.

When a canister 62 full of sampled cuttings 67 arrives at the treatment device 52, the control device commands the following sequence: the first trap door 92 opens and the arm 70 seizes the handle 66. The first trap door 92 closes, the second trap door 94 opens and the arm 70 empties the two cavities 94, respectively into the tray 80 of the washing station 76 and into the archiving bag 86 of the archiving station 82. The control device then triggers the arm 70 to move the canister 62 to the cleaning station 88 and back to the conveyor 60, and the conveyor to carry the canister 62 back to the outlet 48 once it is detected the canister is on the conveyor 60.

The control device 56 also triggers, for instance simultaneously the archiving station 82 so that it closes the archiving bag 86, tag it with depth interval information, and store it it also triggers the washing station to perform the following operations : the cuttings 67 contained in the tray 80 are rinsed and washed, as described above, before the resulting treated cuttings 107 are poured in a carrier 114 situated at the first end 110 of the transport unit 58. When it is detected that a certain weight of cuttings is in the carrier the control device triggers the transport unit so that said carrier 114 is closed and sent to the second end 130 of the transport unit 58, through the pneumatic tube station 118.

When the arrival of a carrier at the second end 130 of the transport unit, connected to the analysis unit 154, is detected, the tag 115 of the carrier 114 is automatically read to identify the arriving sample. The control device 56 triggers the robotic manipulator so that it opens the carrier 114 and pours the cuttings 107 into the tray of the drying and imaging station 162. The control device 56 then triggers the drying and imaging station 162 when cuttings are detected in the tray. After the drying and imaging operations, the control device triggers the manipulator so that it removes the cuttings 107 from the outlet 164 and pours a suitable amount of cuttings into the two grinding canisters 166, 168, The control device then triggers the automated dispenser 174 so that it adds a wax binder into one 166 of the canisters. The control device then triggers the manipulator so that it picks up each grinding canister 166,168 separately, closes canister and inserts said canister Into the ball mill 176, which is energized for a predefined amount of time by the control device to grind the sample into a powder and mix the powder and wax in one 166 of the canisters.

After the certain amount of time, the control device triggers the manipulator so that it pours the contents of the wax-free canister 168 into an XRD sample holder. When a certain weight or volume is detected in the sample holder the control device triggers the XRD analyser so that it performs the analysis operation. The control device 56 also commands the manipulator so that the mixture of powder and wax of the other canister 166 is brought to the pellet press 178 triggered to press the powder into a pellet The control device then commands the manipulator so that said pellet is placed in the XRF analyzer, where the analysis is carried out after triggering of the XRF analyzer by the control device. The control device may trigger the transport device pelletizer, analyzers, etc., so that some operations are performed simultaneously The control device then commands the manipulator so that it brings the empty canisters 166, 168 to the cleaning, such as air cleaning, station 184 where said canisters are cleaned for re-use.

While the XRD and XRF analyzers are operating, the manipulator may be commanded to take care of a new carrier 114 arriving at the second end 130 of the transport unit 58.

After the XRD and XRF analyses are carried out, the manipulator is triggered by the control device to remove the samples from the respective analyzers. The data provided by the analyzers 162, 180, 182 are collected by the control device 56, along with the identification of the corresponding sample.

An automated device 10 according to the embodiments described above is easily relocated along with the installation 12 when the borehole is changed. Such an automated device is easily installed in onshore and off-shore platforms.

In view of the entirety of the present disclosure, including the figures, a person skilled in the art should appreciate that they may readily use the present disclosure as a basis for designing or modifying other processes and structures for carrying out the same uses and/or achieving the same aspects introduced herein. A person skilled In the art should also realize that such equivalent constructions do not depart from the spirit and scope of the present disclosure, and that they may make various changes, substitutions and alterations herein without departing from the spirit and scope of the present disclosure. For example, although the preceding description has been described herein with reference to particular means, materials and embodiments, it is not intended to be limited to the particulars disclosed herein; rather, it extends to functionally equivalent structures, methods, and uses, such as are within the scope of the appended claims.

For instance, the treatment unit is optional and the analysis unit may comprise only one analyzer, It may also comprise fewer stations than what has been disclosed in the disclosure. The analysis unit may comprise other analysers for analysing the prepared samples Instead of, after, before or in parallel with XRD or XRF, such a DRIFTS (Diffuse Reflectance Infrared Fourier Transform Spectroscopy), or LIBS (Laser Induced Breakdown Spectroscopy). The device may also comprise additional analyzers for which the control device commands the insertion of cuttings and operation without previous grinding, such as calcimeter, fluoroscope or X-ray analyzers, Scanning Electron Microscope (SEM).

The device may comprises cleaning stations for cleaning every part of the devices, such as tray, canister, containers, etc. that are in contact with the cuttings and may be reused in the device. The device may also comprise buffers for storing the samples between two different units. The preparation apparatus may also comprise an archiving unit for archiving at least part of cleaned and dried cuttings.

The device may also comprise an interface with a user for displaying the operations carried out by the automated device. It may also comprise a "manual mode" in case of a problem preventing the control device from performing the sequence (for instance, failure of a unit) that enables the user to command one or several of the units via the interface.

## Claims

1. An automated device (10) for analyzing drilling cuttings, said device comprising :
- a sampler (50) for sampling drilling cuttings (47) ; and
- an analysis unit (54,154) for analyzing the treated cuttings (107) ;
wherein the analysis unit comprises:
- a preparation apparatus (144, 176, 178) able to grind the treated cuttings into a powder and subsequently to conform said powder into an analysis sample (145); and
- at least one analyzer (148, 180, 182) ;
and wherein the automated device comprises at least a transport unit (146, 160) able to move the analysis sample from the sampler (50) to the at least one analyzer
and a control device configured to trigger at least an operation of each of the units according to a predetermined sequence.

2. An automated device according to claim 1, wherein the control device is configured to trigger at least an operation of at least a predetermined unit in function of a state of at least one of the units and/or of an external parameter.

3. An automated device according to any one of the preceding claims, wherein the transport unit comprises a robotic manipulator (160, 158) able to insert the sampled cuttings (107) into the preparation apparatus (176, 178) and/or to remove the analysis sample from the at least one analyzer (980, 182).

4. An automated device according to the preceding claim, wherein the analysis unit (154) comprises shelves (156) on which are arranged the preparation apparatus (176, 178) and the at least one analyzer (180, 182), the robotic manipulator also comprising a gripper assembly (160) and a gantry (158) on which the gripper assembly is able to move between the shelves.

5. An automated device according to any one of the preceding claims, wherein the at least one analyzer is an X-ray analyzer, a spectrometer or a TOC analyzer.

6. An automated device according to the preceding claim, wherein the X-ray analyzers (148, 180, 182) is chosen among an X-ray diffraction analyzer (180) and an X-ray fluorescence analyzer (182).

7. An automated device according to claim 5, wherein the spectrometer is chosen among a DRIFTS (Diffuse Reflectance Infrared Fourier Transform Spectrometry) spectrometer, a LIBS (Laser Induced Breakdown Spectrometry) spectrometer, a Raman spectrometer, and a ATR (Attenuated Total Reflectance) spectrometer.

8. An automated device according to any one of the preceding claims, wherein the preparation apparatus (144,174) is able to add a binder to the cutting or to the powder to prepare the analysis sample (145) for analysis.

9. An automated device according to the preceding claim, wherein the preparation apparatus includes a mixer for mixing the binder and cutting or powder and/or a pelletizer.

10. An automated device according to any of the preceding claims, wherein the transport unit (58) comprises at least a pneumatic tube transfer apparatus (116) and/or at least a conveyor.

11. A drilling installation (12) for drilling a wellbore, wherein the drilling Installation comprises:
- a shale shaker (45) situated at the exit of the wellbore for separating a drilling fluid exiting the wellbore and drilling cuttings contained in the drilling fluid,
- an automated device (10) for analyzing drilling cuttings according to any one of the preceding claims, situated at the outlet of the shale shaker.
